# EUROPEAN PATENT SPECIFICATION

(11) **EP 1 846 005 B1**
(45) Date of publication and mention of the grant of the patent: **27.08.2014**
(21) Application number: 06718460.6
(22) Date of filing: 13.01.2006
(51) Int. Cl.: A61K 31/055, A61K 31/137, A61K 31/167, A61K 31/221, A61K 31/245, A61K 31/40, A61K 31/445, A61K 31/46, A61K 31/47, A61K 31/535, A61K 31/727, A61K 31/728, A61K 31/737, A61K 9/00, A61K 45/06, A61K 9/08, A61P 13/02

(54) **Compositions for treating lower urinary tract**
Zusammensetzungen zur Behandlung der unteren Harnwege
Compositions pour traiter une voie urinaire inférieure

(30) Priority: 14.01.2005 US 643885 P; 19.12.2005 US 752287 P
(43) Date of publication of application: 24.10.2007
(73) Proprietor: Urigen, Inc., San Francisco CA 94109 (US)
(72) Inventor: FLASHNER, Michael, San Francisco, California 94109 (US); FRANKLIN, Amie, San Francisco, California 94109 (US); GARNER, William, San Francisco, California 94109 (US); PARSONS, C., San Francisco, California 94109 (US); SEMBER, Michael, San Francisco, California 94109 (US)
(74) Representative: Winter, Brandl, Fürniss, Hübner, Röss, Kaiser, Polte - Partnerschaft mbB
(86) International application number: PCT/US2006/001388
(87) International publication number: WO 2006/076663

(56) References cited:
- WO-A-2005/072751
- US-A- 6 083 933
- US-B2- 6 648 863
- PARSONS C L: "Successful downregulation of bladder sensory nerves with combination of heparin and alkalinized lidocaine in patients with interstitial cystitis" UROLOGY, BELLE MEAD, NJ, US, vol. 65, no. 1, 21 January 2005 (2005-01-21), pages 45-48, XP004724790 ISSN: 0090-4295
- PARSONS C LOWELL: "Current strategies for managing interstitial cystitis" EXPERT OPINION ON PHARMACOTHERAPY, ASHLEY, LONDON, vol. 5, no. 2, 1 February 2004 (2004-02-01), pages 287-293, XP008097859 ISSN: 1465-6566
- DELL JEFFREY R ET AL: "Multimodal therapy for interstitial cystitis" JOURNAL OF REPRODUCTIVE MEDICINE, IL, vol. 49, no. 3, Suppl. S, 1 March 2004 (2004-03-01), pages 243-252, XP008097860 ISSN: 0024-7758
- PARSONS C L: "Evidence-based strategies for recognizing and managing IC" CONTEMPORARY UROLOGY, ADVANSTAR COMMUNICATIONS, US, 1 February 2003 (2003-02-01), pages 22-35, XP008097855 ISSN: 1042-2250
- LUKBAN J C: "Current status in the pharmacological management of interstitial cystitis" EXPERT OPINION ON PHARMACOTHERAPY, ASHLEY, LONDON, vol. 4, no. 11, 1 January 2003 (2003-01-01), pages 1967-1975, XP008097868 ISSN: 1465-6566
- LUKBAN J C ET AL: "Interstitial cystitis and pelvic floor dysfunction: A comprehensive review" PAIN MEDICINE 2001 US, vol. 2, no. 1, 2001, pages 60-71, XP002501826 ISSN: 1526-2375
- LUKBAN J C ET AL: "CURRENT MANAGEMENT IN INTERSTITIAL CYSTITIS" UROLOGIC CLINICS OF NORTH AMERICA, SAUNDERS CO., LONDON, GB, vol. 29, no. 3, 1 August 2002 (2002-08-01), pages 649-660, XP009051896 ISSN: 0094-0143
- WHITMORE K E: "SELF-CARE REGIMENS FOR PATIENTS WITH INTERSTITIAL CYSTITIS" UROLOGIC CLINICS OF NORTH AMERICA, SAUNDERS CO., LONDON, GB, vol. 21, no. 1, 1 February 1994 (1994-02-01), pages 121-130, XP009037042 ISSN: 0094-0143
- DASGUPTA ET AL: "ELECTROMOTIVE DRUG ADMINISTRATION OF LIDOCAINE TO ANESTHETIZE THE BLADDER BEFORE INTRAVESICAL CAPSAICIN" JOURNAL OF UROLOGY, BALTIMORE, MD, US, vol. 159, no. 6, 1 June 1998 (1998-06-01), pages 1857-1861, XP005566059 ISSN: 0022-5347

## Description

### BACKGROUND OF THE INVENTION

This invention is directed to superior buffered formulations and kits including the superior buffered formulations for treating lower urinary tract symptoms and disorders. In particular superior buffered formulations have demonstrated improvement for treating lower urinary tract symptoms of patients experiencing severe pain and/or urgency of the bladder and associated areas of the lower urinary tract. In particular improved treatment methods involve novel intravesicular formulations for bladder instillations for treating, ameliorating, or preventing any one or more pelvic symptoms of pain, urinary urge, urinary frequency, or incontinence.

A large number of diseases and conditions occur in the lower urinary tract and are associated with one or more pelvic symptoms of pain, urge, frequency, or incontinence. In gynecologic patients, pelvic pain is referred to as chronic pelvic pain and may be of unknown origin or may be related to bacterial cystitis, fungal/yeast cystitis, vulvar vestibulitis, vulvodynia, dysparenunia, endometriosis. Regardless of the perceived source of pelvic pain, in many cases the actual source of pain may be the bladder and/or the lower urinary tract. Frequency and urge together encompass the symptoms of overactive bladder. Overactive bladder may also be associated with incontinence, particularly urge incontinence.

In both male and female patients that are treated with cytotoxic therapies for cancer, this may result in any one or more lower urinary tract symptoms of pelvic pain, urge, frequency or incontinence. Localized radiation therapy to the pelvis which occurs due to bladder, cervical, ovarian, rectum, colon, vagina/vulva or prostate cancer therapy, may result in damaging the epithelium of the bladder wall leading to one or more of lower urinary tract symptoms of pain, urge, and/or frequency. Cytotoxic cancer chemotherapy, most notably cyclophosphamide and ifosfamide treatment for breast cancer patients (male and female) may also lead to the same series of symptoms.

In male patients, any one or more lower urinary tract pelvic symptoms of pelvic pain, urge, frequency or incontinence is observed in patients with prostatitis, chronic pelvic pain syndrome, urethral syndrome, or overactive bladder.

There are no specific treatments for lower urinary tract pelvic pain and instead patients are prescribed oral NSAIDS such as aspirin or acetaminophen. For severe chronic pain, some subjects rely on oral and/or transdermal narcotics which typically results in an irreversible worsening of symptoms.

For the symptoms of urinary urge and frequency, also termed overactive bladder, oral anticholinergic drugs such as detroloxybutynin chloride (Ditropan XL®) and tolterodine (Detrusitol®, Detrol LA®) reduce the contraction of the smooth muscle of the bladder wall. However, these drugs do not treat the underlying cause of the problem. Additionally, these drugs may result in side effects such as dry mouth, constipation, headache, blurred vision, hypertension, drowsiness, and urinary retention in approximately 50%. The benefits of these drugs do not appear to overcome their risks/detriments since only 20% of patients refill their prescriptions.

There is one agent, Mesnex® (mesna) that is used for the prevention of hemorrhagic cystitis due to ifosfamide treatment in cancer patients. This agent is a detoxifying agent and binds and detoxifies the cancer drug. The drug does not treat acute pain and actually results in very high frequency of adverse events (all AEs for IV = 85%, for oral = 89%), most notable adverse events are nausea, vomiting, and constipation.

Although heparinoid-based therapy (heparin; the oral agent pentosan polysulfate sodium [PPS]) is an effective treatment for interstitial cystitis (IC), patients may require several months of therapy or more before they experience relief of pain and urgency/frequency. (P.M. Hanno, "Analysis of Long-Term Elmiron Therapy for Interstitial Cystitis," Urology 49(Suppl 5A): 93-99 (1997)) Heparinoids, which are believed to augment the dysfunctional epithelium that is present in many cases of the disease, take time to reach full effectiveness in reversing the disease process and thereby reducing symptoms. (C. L. Parsons," Epithelial Coating Techniques in the Treatment of Interstitial Cystitis. Urology 49(Suppl 5A):100-104 (1997)). In addition, particularly in severe or long-standing cases of IC, there is significant upregulation of the sensory nerves in the bladder. (T.J. Christmas et al., "Nerve Fibre Proliferation in Interstitial Cystitis," Virchows Archiv. A Pathol. Anat. 416: 447-451 (1990); X. Pang et al., "Increased Number of Substance P Positive Nerve Fibres in Interstitial Cystitis," Br. J. Urol. 75:744-750 (1995); C.A. Buffington & S.A. Wolfe, Jr., "High Affinity Binding Sites for [3H]Substance P in Urinary Bladders of Cats with Interstitial Cystitis," J. Urol. 160:605-611 (1998)). Heparinoids allow natural downregulation of the nerves over time by gradually restoring the barrier function of the mucus and thus preventing further irritation by urinary constituents such as potassium (J.C. Nickel et al., "Randomized, Double-Blind, Dose-Ranging Study of Pentosan Polysulfate Sodium (PPS) for Interstitial Cystitis (IC)," J. Urol. 165(5 Suppl): 67 (2001); C.L. Parsons et al., :"Effect of Pentosan Polysulfate Therapy on Intravesical Potassium Sensitivity," Urology 59: 329-333 (2002)) No currently available IC therapy achieves immediate symptom relief without destroying the nerve endings (T.W. Cannon & M.B. Chancellor, "Pharmacotherapy of the Overactive Bladder and Advances in Drug Delivery," Clin. Obstet. Gynecol. 45: 205-17 (2002); M.B. Chancellor & N. Yoshimura, "Treatment of Interstitial Cystitis," Urology 63(3 Suppl 1): 85-89 (2004); M. Lazzeri et al., "Intravesical Infusion of Resiniferatoxin by a Temporary in Situ Drug Delivery System to Treat Interstitial Cystitis: A Pilot Study," Eur. Urol. 45: 98-102 (2004)) or employing narcotics. Thus there continues to be a need for an IC treatment that offers immediate relief of symptoms and operates directly to downregulate the bladder sensory nerves without any rebound effect.

Intravesical agents have been used for many years as adjuncts to oral treatment regimens or as second-line therapies for IC. One of the most widely used is heparin, which is effective in approximately 50% of patients treated. Heparin is a sulfated polysaccharide that is believed to augment the protective effect of the natural bladder surface mucus. Intravesical heparinoid agents alone, however, do not produce immediate and sustained relief of IC symptoms. Like the oral heparinoids, they take several months to produce symptom relief.

Other treatments have also been tried, with limited success. For example, treatments with dimethylsulfoxide (DMSO), approved for IC in 1977 on the basis of data from uncontrolled trials, can be useful with weekly intravesical instillations for 6 to 8 weeks then every two weeks for 3-12 months for maintenance. However DMSO therapy results in benefit for approximately only 50% of IC patients treated and the treatment takes a long time to reduce symptoms. Furthermore, this therapy causes pain that is unrelieved by local anesthetics by themselves due to their lack of absorption into the bladder wall. Narcotics are given for immediate relief of symptoms however they are only minimally effective. The use of narcotics, of course, carries a significant risk of tolerance and addiction. Some patients benefit from formal 8- to 12-week, one-on-one course of behavior modification. Patients are also advised to avoid potassium-rich foods, particularly citrus fruits, tomatoes, chocolate, and coffee.

Many urologists treat interstitial cystitis patients with their own "home-brew" of drugs by administering the drug(s) or mixtures thereof into the lumen of the bladder. As these procedures are typically done in the office without any quantitative assessment of severity of initial symptoms prior to or subsequent to treatment, there is no scientific rigor in assessing the benefit of these treatments. Consequently, patients are treated with drugs in their non-approved indications with no real scientific guidance as to whether the patient will benefit from the treatment or not.

Consequently, there is a tremendous need for scientifically-validated and improved treatments that provide immediate relief for treating lower urinary tract symptoms and disorders, particularly those with severe interstitial cystitis. Additionally, these treatments should be based on validated quantitative assessment of benefit, not on wishful thinking which has been the basis of urologists "home-brew" treatments that are not assessed quantitatively. There is a particular need for improved treatments and compositions for use in those treatments that provide immediate relief and do not require several months until the patients experience relief.

Already known is a treatment of the lower urinary tract disorder interstitial cystitis (IC) (Parsons, CL "Current strategies for managing interstitial cystitis" Expert Opinion of Pharmacotherapy, 5(2), 287-293, 2004), comprising instillation of a solution containing 10000-40000 U of heparin dissolved in 10 ml buffered sodium chloride, 10 ml 1% lidocaine or 16 ml 2% lidocaine and 3 ml 8,4% sodium bicarbonate.

Previously, Parsons (Parsons, CL "Evidence-based strategies for recognizing and managing IC" Contemporary Urology, February, pps. 22-35, 2003) published a recipe of three FDA-approved drug components for the treatment of interstitial cystitis, which is a painful bladder disorder of unknown etiology. The components were 80 mg lidocaine (8 ml 1% lidocaine), 40,000 units heparin (4 ml of 10,000 units/ml heparin sodium), and 252 mg bicarbonate (3ml of 8.4% sodium bicarbonate) in a total aqueous volume of 15ml.

An additional limitation of the Parsons approach is that components have to be measured out immediately before use from three separate solutions. In many treatment settings such as clinics or doctor's offices there are neither the pharmaceutical personnel resources qualified to measure out these components from stock solutions or the possibility exists of accidental mis-measurement leading to the potential for incorrect treatment or lidocaine overdose. Additionally, this mixing in a non-sterile environment may result in contamination With an infectious agent or other detrimental component that would be directly instilled in a compromised bladder. Consequently, this invention provides for pre-measured kits and prefilled vials of the formulation of the invention that prevents these point-of-care problems.

An aspect of the previous formulation by Parsons is that it contained multiple components and with any solution or equation with multiple variables, it cannot be clear what portion of the each component contributes to the effect. Accordingly, there is a need for an improvement in the formulation that would maximize the effect of each component.

### SUMMARY OF THE INVENTION

The present invention embodies improved compositions for treating pelvic disorders according to claims 1 to 3. The improved intravesicular formulations for pelvic disorders are usable for treating or preventing of bacterial cystitis, fungal/yeast cystitis, vulvar vestibulitis, vulvodynia, dysparenunia, and endometriosis in women; prostatitis, chronic pelvic pain syndrome, or urethral syndrome in men; and radiation-induced cystitis, chemotherapy-induced cystitis, or overactive bladder in men or women.

The composition comprises:
(1) 160 mg lidocaine per unit dose;
(2) 40,000 units of heparin per unit dose;
(3) 336 mg sodium bicarbonate per unit dose; and
(4) 20 mg sodium chloride per unit dose;
such that, in a final unit dose volume of 12 ml, lidocaine is present at 46 mM, heparin is present at 3333 units/ml, sodium bicarbonate is present at 0.33 M, and sodium chloride is present at about 28.5 mM.

In another alternative, the composition comprises:
(1) 200 mg lidocaine per unit dose;
(2) 40,000 units of heparin per unit dose;
(3) 336 mg sodium bicarbonate per unit dose; and
(4) 20 mg sodium chloride per unit dose;
such that, in a final dose volume of 13 ml, lidocaine is present at 53 mM, heparin is present at 3077 units/ml, sodium bicarbonate is present at 0.305 M, and sodium chloride is present at 26.3 mM.

In still another alternative, the composition comprises:
(1) 240 mg lidocaine per unit dose;
(2) 40,000 units of heparin per unit dose;
(3) 336 mg sodium bicarbonate per unit dose; and
(4) 20 mg sodium chloride per unit dose;
such that, in a final dose volume of 14 ml, lidocaine is present at 64 mM, heparin is present at 2857 units/ml, sodium bicarbonate is present at 0.28 M, and sodium chloride is present at 24.4 mM.

### BRIEF DESCRIPTION OF THE DRAWINGS

The following invention will become better understood with reference to the specification, appended claims, and accompanying drawings, where:

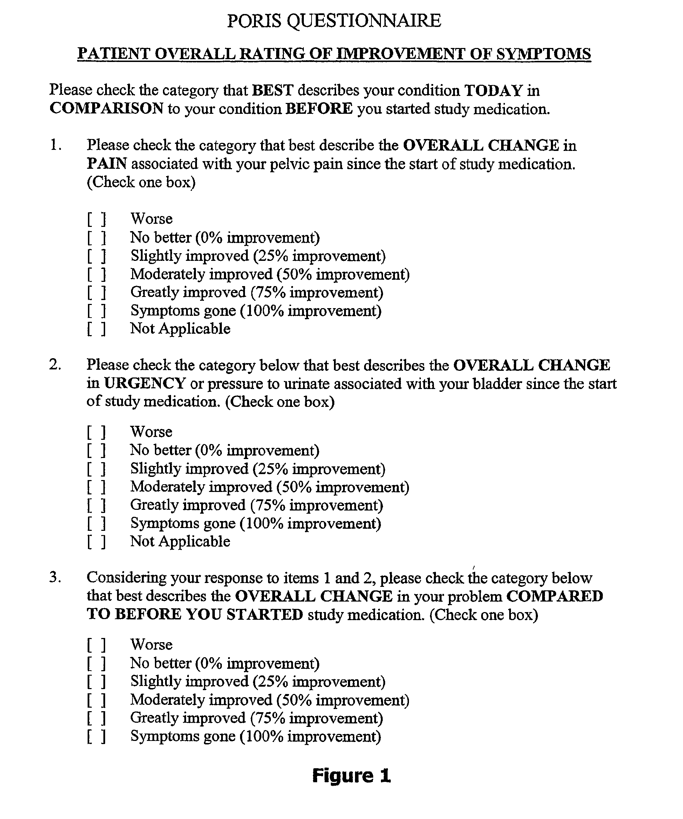

Figure 1 is the validated PORIS questionnaire, "Patient Overall Improvement of Symptoms" that has been used for the approval of oral Elmiron^{®} (pentosan polysulfate sodium) with the U.S. FDA. The primary endpoint for that approval and for the study in the current invention was "Moderately improved"; i.e., 50% or greater improvement in overall change in combined symptoms of pain and/or urgency since the start of therapy.

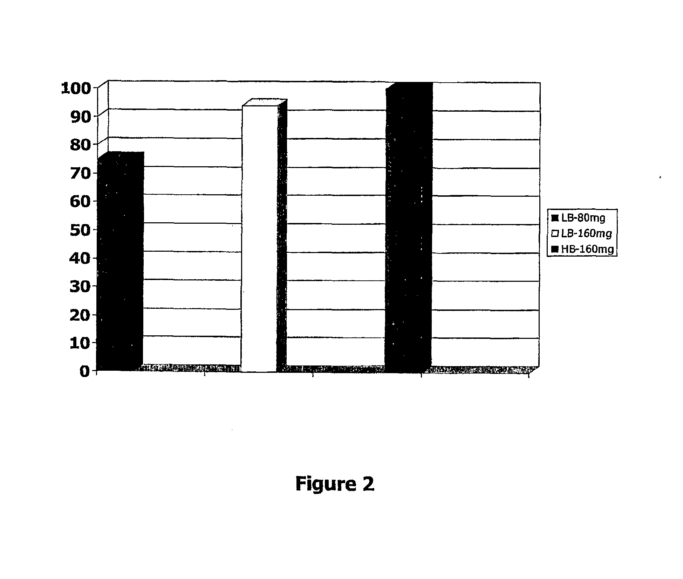

Figure 2 is a graph comparing the PORIS results on a preferred composition according to the present invention, with 160 mg of lidocaine per unit dose and 0.33 M sodium bicarbonate as buffer ("HB-160 mg"), a superior buffered formulation, versus a low buffer formulation (sodium bicarbonate at 0.2 M) with 160 mg lidocaine ("LB-160 mg") and low buffer with 80 mg lidocaine ("LB-80 mg"). 26 patients were treated with HB-160 mg, 35 were treated with LB-160 mg and 47 were treated with LB-80 mg. A positive result was if a patient exhibited "moderately improved" combined symptoms of pain and urgency - PORIS question 3. Furthermore, 6 patients were on chronic narcotic use for greater than 6 months, and 4/6 of these patients experienced 100% relief on PORIS scale and the remaining 2/6 experienced 75% relief on PORIS scale. Additionally, the patients treated with HB-160 mg had fairly severe symptoms of pain and/or urge with 15/26 (58%) had PUF scores >20. All patients had greater than 15 PUF score.

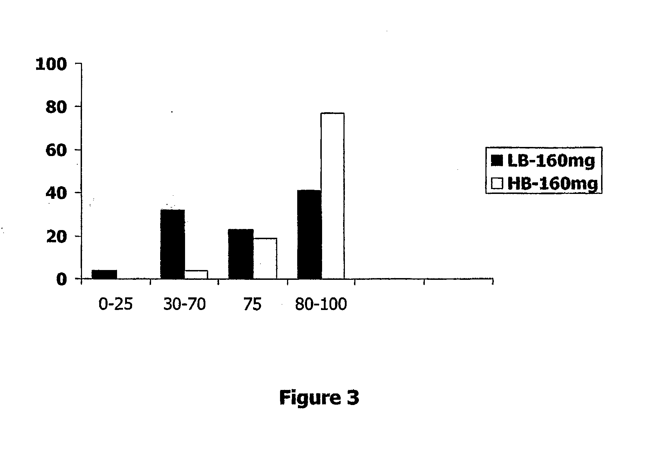

Figure 3 is a graph comparing the relative distribution of PORIS scores between patients administered the study drug of the current invention, HB-160 mg versus LB-160 mg. More patients experienced a 100% improvement in symptoms (>75% of patients) versus the older formulation, LB-160 mg in which only about 40% of patients experienced a 100% improvement in combined symptoms of pain and urgency. Furthermore, with the HB-160 mg formulation patients had >25% improvement in symptoms of pain and urgency whereas with the LB-160 mg, a small group of patients was essentially unresponsive (0-25% improvement in symptoms). Consequently, validated PORIS patient responses clearly demonstrate superiority of the formulation of the present invention over LB-160 mg with a statistically significant P value of 0.009.

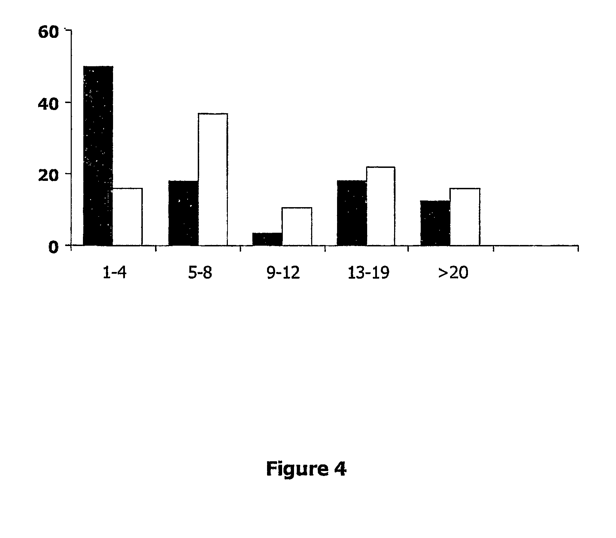

Figure 4 is a graph of the duration of relief of combined symptoms of pain and urgency of the formulation according to the present invention, HB-160 mg. Study patients were followed up 24 hr after treatment in the clinic with study drug and asked how long of a benefit from the treatment they received. The half-life of lidocaine when used for its anesthetic effect alone is 1.5 hr. For the patients treated with the HB-160 mg formulation, the distribution of time was much longer and median duration was 7 hours, significantly longer than predicted than the half-life of lidocaine. In comparison, the median duration of benefit of LB-160 mg was only 4 hours.

### DETAILED DESCRIPTION OF THE INVENTION

The invention is a pharmaceutical composition for use in the treatment or prevention of treating, or preventing a lower urinary tract disorder according to claims 1 to 3.

For a composition according to the present invention, comprising lidocaine, heparin, sodium bicarbonate, and sodium chloride, amounts and concentrations, as well as the resulting molarities where appropriate, are shown below in Table 1.

**TABLE 1**

| **COMPONENTS OF PREFERRED COMPOSITION** | | | |
|---|---|---|---|
| **Component** | **Amount** | **Conc.** | **Molarity** |
| **Lidocaine** | 160 mg | 1.33% | 46 mM |
| **Heparin** | 40,000 U | 3,333 U/ml | |
| **Sodium Bicarbonate** | 336 mg | 2.8% | 0.33 M |
| **Sodium Chloride** | 20 mg | 0.2% | 28.5 mM |
| **Total Volume** | 12 ml | | |

For another composition according to the present invention, comprising lidocaine, heparin, sodium bicarbonate, and sodium chloride, amounts and concentrations, as well as the resulting molarities where appropriate, are shown below in Table 2.

**TABLE 2**

| **COMPONENTS OF PREFERRED COMPOSITION** | | | |
|---|---|---|---|
| **Component** | **Amount** | **Conc.** | **Molarity** |
| **Lidocaine** | 200 mg | 1.53% | 53 mM |
| **Heparin** | 40,000 U | 3,077 U/ml | |
| **Sodium Bicarbonate** | 336 mg | 2.58% | 0.305 M |
| **Sodium Chloride** | 20 mg | 0.185% | 26.3 mM |
| **Total Volume** | 13 ml | | |

For yet another composition according to the present invention, comprising lidocaine, heparin, sodium bicarbonate, and sodium chloride, amounts and concentrations, as well as the resulting molarities where appropriate, are shown below in Table 3.

**TABLE 3**

| **COMPONENTS OF PREFERRED COMPOSITION** | | | |
|---|---|---|---|
| **Component** | **Amount** | **Conc.** | **Molarity** |
| **Lidocaine** | 240 mg | 1.71% | 64 mM |
| **Heparin** | 40,000 U | 2,857 U/ml | |
| **Sodium Bicarbonate** | 336 mg | 2.4% | 0.28 M |
| **Sodium Chloride** | 20 mg | 0.17% | 24.4 mM |
| **Total Volume** | 14 ml | | |

The lower urinary tract disorder is bacterial cystitis, fungal/yeast cystitis, vulvar vestibulitis, vulvodynia, dysparenunia, and endometriosis in women; prostatitis, chronic pelvic pain syndrome, or urethral syndrome in men; and radiation-induced cystitis, chemotherapy-induced cystitis, or overactive bladder in men or women. A significant lower urinary tract disorder treatable by the use of compositions and methods according to the present invention is interstitial cystitis (IC), which is more frequently diagnosed in women, but is now being diagnosed more frequently in men as well. Another significant lower urinary tract disorder treatable by the use of compositions and methods according to the present invention is radiation-induced cystitis.

Compositions according to the present invention can include other ingredients. It is possible to include a compound that enables persistence of the composition to the surface of the bladder wall in the composition. A suitable compound that enables persistence of the composition to the surface of the bladder wall is an activatable gelling agent. The addition of an activatable gelling agent that would result in the formation of a gel on the bladder epithelial surface would ensure improved transference of the active drugs (the anesthetic and the bladder coating anionic polysaccharide) to the areas most needing them. In one instance the gelling agent is liquid at room temperature and then upon bladder instillation, would gel at body temperature; in other words, the activatable gelling agent is a thermoreversible gelling agent. This feature of thermoreversible gelation has been observed for Pluronics F127 gel, Lutrol gel (T. Beynon et al., "Lutrol Gel: A Potential Role in Wounds?," J. Pain Symptom Manage. 26: 776-780 (2003)), NASI-containing polymers (N-isopropylacrylamide, ethylmethacrylate, N-acryloxysuccinimide) (T.J. Gao et al., "Synthetic Thermoreversible Polymers Are Compatible with Osteoinductive Activity of Recombinant Human Bone Morphogenetic Protein 2," Tissue Eng. 8: 429-440 (2002); T. Gao & U.H. Uludag, "Effect of Molecular Weight of Thermoreversible Polymer on in Vivo Retention of rhBMP-2," J. Biomed. Mater. Res. 57: 92-100 (2001)), xyloglucan sols of 1-2% (S. Miyazaki et al., "Thermally Reversible Xyloglucan Gels as Vehicles for Rectal Drug Delivery," J. Control Release 4: 75-83 (1998)), graft copolymers of pluronic and poly(acrylic acid), pluronic-chitosan hydrogels, and a [Poly(ethylene glycol)-Poly[lactic acid-co-glycolic acid]-Poly(ethylene glycol)) (PEG-PLGA-PEG) polymer (P. Tyagi et al., "Sustained Intravesical Drug Delivery Using Thermosensitive Hydrogel," Pharm. Res. 21: 832-837 (2004)). In general, these polymers are sols at room temperature but form gels at body temperature, about 37°C. Other activatable gelling agents are known in the art.

In yet another alternative, the composition can further comprise an antibacterial agent or an antifungal agent to treat bacterial or fungal cystitis. Suitable antibacterial agents include, but are not limited to: (1) sulfonamides such as sulfanilamide, sulfadiazine, sulfamethoxazole, sulfisoxazole, sulfamethizole, ; sulfadoxine, and sulfacetamide; (2) penicillins such as methicillin, nafcillin, oxacillin, cloxacillin, dicloxacillin, ampicillin, amoxicillin, bacampicillin, carbenicillin, ticarcillin, mezlocillin, and piperacillin; (3) a combination of trimethoprim plus sulfamethoxazole; (4) quinolones such as nalidixic acid, cinoxacin, norfloxacin, ciprofloxacin, orfloxacin, sparfloxacin, lomefloxacin, fleroxacin, pefloxacin, and amifloxacin; (5) methenamine; (6) nitrofurantoin; (7) cephalosporins such as cephalothin, cephazolin, cephalexin, cefadroxil, cefamandole, cefoxatin, cefaclor, cefuroxime, loracarbef, cefonicid, cefotetan; ceforanide, cefotaxime, cefpodoxime proxetil, ceftizoxime, ceftriaxone, cefoperazone, ceftazidime, and cefepime; (8) carbapenems such as imipenem, meropenem, and aztreonam; (9) aminoglycosides such as netilmycin and gentamicin; (10) tetracyclines such as tetracycline, oxytetracycline, demeclocycline, minocycline, doxycycline, and chlortetracycline; and (11) macrolides such as erythromycin, clarithromycin, and azithromycin. Antifungal agents include amphotericin B, itraconazole, ketoconazole, fluconazole, miconazole, and flucytosine. Other suitable antibacterial agents and antifungal agents are known in the art.

In yet another alternative, the compositions according to the present invention can include a vasoconstrictor. The purpose of a vasoconstrictor is to constrict the blood vessels locally at or near the site of administration to ensure that the composition has its maximum effect at or near the site of administration. A particularly preferred vasoconstrictor is epinephrine. Typically, if the anesthetic is lidocaine, the ratio of epinephrine to lidocaine is from about 1:1000 to about 1: 200,000. Preferably, if the anesthetic is lidocaine, the ratio of epinephrine to lidocaine is about 1:100,000.

Preferred amounts and concentrations are as described above. Particularly preferred amounts and concentrations are described in Table 1, Table 2, and Table 3.

Accordingly, a composition according to the present invention comprises:
(1) 160 mg lidocaine per unit dose;
(2) 40,000 units of heparin per unit dose;
(3) 336 mg sodium bicarbonate per unit dose; and
(4) 20 mg sodium chloride per unit dose;
such that, in a final dose volume of 12 ml, lidocaine is present at 46 mM, heparin is present at 3333 units/ml, sodium bicarbonate is present at 0.33 M, and sodium chloride is present at about 28.5 mM.

Accordingly, another composition according to the present invention comprises:
(1) 200 mg lidocaine per unit dose;
(2) 40,000 units of heparin per unit dose;
(3) 336 mg sodium bicarbonate per unit dose; and
(4) 20 mg sodium chloride per unit dose;
such that, in a final dose volume of 13 ml, lidocaine is present at 53 mM, heparin is present at 3077 units/ml, sodium bicarbonate is present at 0.305 M, and sodium chloride is present at 26.3 mM.

Accordingly, yet another composition according to the present invention comprises:
(1) 240 mg lidocaine per unit dose;
(2) 40,000 units of heparin per unit dose;
(3) 336 mg sodium bicarbonate per unit dose; and
(4) 20 mg sodium chloride per unit dose;
such that, in a final dose volume of 14 ml, lidocaine is present at 64 mM, heparin is present at 2857 units/ml, sodium bicarbonate is present at 0.28 M, and sodium chloride is present at 24.4 mM.

Typically; compositions according to the present invention are instilled into the bladder, a route of administration that is referred to as intravesical administration. Typically, this is performed by catheterization. Suitable catheters, and methods for installing the catheters, delivering the composition, and removing the catheters are known in the art and need not be described further here. Typical catheters are made of elastic, elastic web, rubber, glass, metal, or plastic.

Typically, compositions according to the present invention are administered 3-7 times weekly for three weeks or more or on an "as-needed" basis to control acute symptoms of pain and urgency of the lower urinary tract. The treading physician can adjust the frequency and duration of treatment according to the response of the patient, the severity of the symptoms, the degree of pain and discomfort subjectively experienced by the patient, and other factors such as the results of histological tests.

Additionally, still other ingredients can be included in compositions according to the present invention. Such ingredients can include, for example, a coloring agent, a preservative, an antioxidant, a chelating agent, and other ingredients typically used in pharmaceutical formulations. For example, a non-toxic, non-allergenic, non-sensitizing coloring agent can be added for convenience in dispensing and administering the composition. Such coloring agents are well known in the art and are used in many liquid pharmaceutical compositions. Suitable examples of preservatives include, for example, parabens, chlorobutanol, phenol, sorbic acid, or thimerosal.

If sterilization of the composition is required, it is typically performed by filtration. Other sterilization methods are known in the art.

The exact formulation and dosage can be chosen by the individual physician in view of the patient's condition. (See e.g. Fingl et al., in The Pharmacological Basis of Therapeutics, 1975, Ch. 1 p. 1). It should be noted that the attending physician would know how to and when to terminate, interrupt, or adjust administration due to toxicity, or to organ dysfunctions. Conversely, the attending physician would also know to adjust treatment to higher levels if the clinical response were not adequate (precluding toxicity). The magnitude of an administered dose in the management of the disorder of interest will vary with the severity of the condition to be treated, the general condition of the urinary tract, including the bladder and urethra, and the existence of other conditions affecting the urinary tract, such as infections, inflammation, or allergic reactions. The severity of the condition may, for example, be evaluated, in part, by standard prognostic evaluation methods, including the ones described below. Further, the dose, and perhaps the dose frequency, will also vary according to the age, body weight, and response of the individual patient. A program comparable to that discussed above may be used in veterinary medicine.

One prognostic evaluation method is the Pelvic Pain and Urgency/Frequency Patient Symptom Scale (PUF Scale) (Parsons, et al. Urology 60:573-578 (2002)). The PUF Scale is a self-administered questionnaire that can be completed by the patient in less than 5 minutes. It contains questions that elicit and quantify urinary frequency and/or urgency (if any), pelvic pain, and/or pain associated with sexual activity. The result is a single numeric score from 0 through 35. The higher the PUF score, the greater the likelihood that the individual has interstitial cystitis, one of the conditions referred to above (Parsons, et al. Urology 60:573-578 (2002)); for this reason, the PUF can be useful in distinguishing interstitial cystitis from other disorders during the process of diagnosis.

Another prognostic test that is useful is the Potassium Sensitivity Test (PST). Bladder epithelial permeability and urinary potassium appear to play a key role in the development of many cases of the disease [Parsons, et al. J Urol 159:1862-1867 (1998)]. In the healthy bladder, a mucus layer containing glycosaminoglycans (GAGs) forms a barrier that prevents urine and its contents from leaking through the urothelium and damaging the underlying nerves and muscle [Lilly and Parsons, Surg Gynecol Obstet 171:493-496 (1990)]. Most individuals with IC have an epithelial dysfunction that renders the urothelium abnormally permeable. As a result, potentially harmful substances in urine are allowed to leak through the epithelium and penetrate the bladder muscle. Potassium, which occurs in high concentrations in normal urine, does not damage or penetrate a healthy urothelium but is highly toxic to tissues such as the bladder musculature. The depolarization of sensory nerves in the bladder muscle by potassium could produce the symptoms of IC as well as cause its progression. A growing body of data supports this hypothesis. On the basis of this model of IC pathogenesis, the Potassium Sensitivity Test (PST) was developed to test for the presence of abnormal bladder epithelial permeability. The use of the PST is described, for example in Parsons, et al. Urology 57:428-33 (2001), Parsons and Albo, J Urol 168:1054-1057 (2002); Koziol, Urol Clin North Am 21:7-71 (1994). The PST has been positive in 78% of those IC patients tested, providing considerable evidence most IC patients have a urothelial permeability defect, and that a positive PST is a valid indicator of the presence of IC [Parsons, et al. Urology 57:428-33 (2001); Parsons and Albo, J Urol 168:1054-1057 (2002).

Other prognostic tests can be used to determine the existence and severity of the conditions described above. These tests are known to clinicians and others of ordinary skill in the art.

The invention is illustrated by the following Examples. These Examples are included for illustrative purposes only and are not intended td limit the invention.

### EXAMPLE 1

Two studies were undertaken on patients with symptoms of pelvic pain and urgency. Patient symptom severity was determined by the PUF questionnaire, Pelvic Pain, Urgency/Frequency questionnaire. Patients were treated with an intravesical instillation of formulations described below and then therapeutic efficacy was assessed within 30 min to 1 hour by the PORIS questionnaire as shown in Figure 1, Patient Overall Rating of Improvement of Symptoms. Patients were followed up 24 hr after treatment and asked about the duration of benefit, if any, of the treatment.

The composition of the formulations is provided in Table 4 below. The HB-160 mg and the LB-160 mg formulations are provided as part of the current invention.

**Table 4**

| **Component** | **HB-160 mg** | **LB-160 mg** | **LB-80 mg** |
|---|---|---|---|
| **Lidocaine HCl** | 46 mM (160 mg) | 37 mM (160 mg) | 18.5 mM (80 mg) |
| **Heparin Sodium** | 3,333 u/ml | 2,666 u/ml | 2,666 u/ml |
| **Sodium Bicarbonate** | 0.33 M | 0.20M | 0.20 M |
| **Sodium Chloride**** | 28.5 mM | 77.5 mM | 77.5 mM |
| **Total Volume** | 12 ml | 15 ml | 15 ml |

| | | | |
|---|---|---|---|
| ** The sodium chloride is provided as a separate component, however, please note that additional sodium ions are also supplied by the sodium salts of bicarbonate and heparin and additional chloride ions are provided by the anion salt of lidocaine and this additional "sodium chloride" is not corrected for in the table. | | | |

In the first study, two groups of patients were treated with two different formulations. One designated here low buffer with 80 mg lidocaine, LB-80 mg, and the other low buffer with 160 mg lidocaine, LB-160 mg that is provided by this invention. After one instillation of LB-80 mg, 35 of 47 patients, 75% experienced significant immediate relief of both pain and urgency as defined by a 50% or greater improvement "moderately improved" on the PORIS scale. In another arm of this study 33 of 35 patients, 94%, experienced significant immediate relief of both pain and urgency as defined by a 50% or greater improvement "moderately improved" on the PORIS scale. A followup phone call was used to monitor duration of effect in patients that had received one instillation of LB-160 mg, and 50% of these patients experienced at least 4 hours of symptom relief.

In the second study, the formulation, HB-160 mg provided by this invention was tested on 26 patients. Of the patients treated with HB-160 mg, most had fairly severe symptoms of pain and/or urge with 15/26 (58%) having PUF scores >20; all patients had PUF scores greater than 15. As shown in Figure 2, 100% of patients experienced significant immediate relief of both pain and urgency as defined by a 50% or greater improvement "moderately improved" on the PORIS scale. Notably, 6 patients were on chronic narcotic use for greater than 6 months, and 4/6 of these patients experienced 100% relief on PORIS scale and the remaining 2/6 experienced 75% relief on PORIS scale. In contrast, the LB-160 mg formulation has only provided limited to no benefit to this severe class of patients experiencing severe chronic pelvic pain, urgency and frequency.

Seemingly, the difference between 94% of patients experiencing "moderate improvement" with the LB-160 mg formulation versus the 100% of patients experiencing "moderate improvement" with the HB-160 mg may seem to be a minor increase in efficacy. However, as shown in Figure 3 the distribution of PORIS scores is significantly shifted to the right with statistically significant P value of 0.009, with nearly 80% of patients treated with HB-160 mg experiencing a 100% improvement in symptoms meaning that their symptoms were gone. In contrast, only a little over 40% of patients treated with LB-160 mg had their "symptoms gone" demonstrating that HB-160 mg had doubled the proportion of patients in the 100% improvement symptom class. Additionally, all patients treated, even those severe interstitial cystitis (IC) patients, experienced some benefit as there were no patients in the "worse", "no better" or "slightly improved" class of symptoms with "slightly improved" equivalent to up to 25% improvement of combined symptoms of pain and urgency.

Another significant difference between HB-160 mg and LB-160 mg was in the duration of relief experienced by patients. Patients were followed up 24-48 hr after their treatment and asked about the duration of relief from symptoms of pain and/or urgency. As previously mentioned, the average duration of relief for patients treated with LB-160 mg was 4 hours, however, the distribution of relief of the patients is graphed in Figure 3 and approximately 50% of patients treated with LB-160 mg only experienced 1-4 hours of relief which is not dramatically longer than the half-life of the anesthetic component lidocaine which has a half-life of 1.5 hr. In contrast, patients treated with HB-160 mg experienced a significant longer average duration of benefit of 7 hours. Only a minority of these patients, approximately 15% experienced 1-4 hours of relief which is very different from the 50% of patients treated with the LB-160 mg.

### EXAMPLE 2

A small study was undertaken on another modification of the formulation designated HB-200 mg. The total lidocaine dose per treatment was increased to 200 mg and the total amount of the other components was altered as shown in the following table 5. A total of 15 patients known to have significant symptoms of pelvic pain and urgency were administered the HB-200 mg solution via an intravesical instillation. All 15 patients experienced a significant reduction in their symptoms of pain and urgency however, one patient did experience a slight headache which is a side-effect of elevated systemic levels of lidocaine. This was not severe and was readily reversible by lowering the lidocaine dosage.

**Table 5**

| **Component** | **HB-200 mg** | **HB-160 mg** | **LB-160 mg** | **LB-80 mg** |
|---|---|---|---|---|
| **Lidocaine HCl** | 53 mM (200 mg) | 46 mM (160 mg) | 37 mM (160 mg) | 18.5 mM (80 mg) |
| **Heparin Sodium** | 3,076 u/ml | 3,333 u/ml | 2,666 u/ml | 2,666 u/ml |
| **Sodium Bicarbonate** | 0.30 M | 0.33 M | 0.20M | 0.20 M |
| **Sodium Chloride**** | 26.3 mM | 28.5 mM | 77.5 mM | 77.5 mM |
| **Total Volume** | 13 ml | 12 ml | 15 ml | 15 ml |

### ADVANTAGES OF THE INVENTION

Improved compositions according to the present invention provide an improved way of treating a number of chronic, hard-to-treat lower urinary tract conditions that affect a large number of people, many of whom have not been diagnosed properly. These improved compositions are directed towards resolving the pathophysiological basis of the conditions by reducing the abnormal permeability of the bladder epithelium and also desensitizing the activity of the nerves involved in the condition. They can be used together with other therapies for symptoms such as pain, if desired, do not cause significant side effects, and are well tolerated.

Moreover, the improved compositions according to the present invention provide rapid relief and do not require extended periods of time, such as several months, to provide relief. This is particularly important and provides a clear advantage over previous treatment.

## Claims

1. A pharmaceutical composition for use in the treatment or prevention of a lower urinary tract disorder comprising:
(a) 160 mg lidocaine per unit dose;
(b) 40,000 units heparin per unit dose;
(c) 336 mg sodium bicarbonate per unit dose; and
(d) 20 mg sodium chloride per unit dose;
such that, in a final unit dose volume of 12 ml, lidocaine is present at 46 mM, heparin is present at 3333 units/ml, sodium bicarbonate is present at 0.33 M, and sodium chloride is present at 28.5 mM, said lower urinary tract disorder being selected from the group consisting of bacterial cystitis, fungal/yeast cystitis, vulvar vestibulitis, vulvodynia, dyspareunia, endometriosis in women, prostatitis, chronic pelvic pain syndrome, urethral syndrome in men, radiation-induced cystitis, chemotherapy-induced cystitis, interstitial cystitis and overactive bladder in men and women.

2. A pharmaceutical composition for use in the treatment of prevention of a lower urinary tract disorder comprising:
(a) 200 mg lidocaine per unit dose;
(b) 40,000 units of heparin per unit dose;
(c) 336 mg sodium bicarbonate per unit dose; and
(d) 20 mg sodium chloride per unit dose;
such that, in a final dose volume of 13 ml, lidocaine is present at 53 mM, heparin is present at 3077 units/ml, sodium bicarbonate is present at 0.305 M, and sodium chloride is present at 26.3 mM, said lower urinary tract disorder being selected from the group consisting of bacterial cystitis, fungal/yeast cystitis, vulvar vestibulitis, vulvodynia, dyspareunia, endometriosis in women, prostatitis, chronic pelvic pain syndrome, urethral syndrome in men, radiation-induced cystitis, chemotherapy-induced cystitis, interstitial cystitis and overactive bladder in men and women.

3. A pharmaceutical composition for use in the treatment or prevention of a lower urinary tract disorder comprising:
(a) 240 mg lidocaine per unit dose;
(b) 40,000 units of heparin per unit dose;
(c) 336 mg sodium bicarbonate per unit dose; and
(d) 20 mg sodium chloride per unit dose;
such that, in a final dose volume of 14 ml, lidocaine is present at 64 mM, heparin is present at 2857 units/ml, sodium bicarbonate is present at 0.28 M, and sodium chloride is present at 24.4 mM, said lower urinary tract disorder being selected from the group consisting of bacterial cystitis, fungal/yeast cystitis, vulvar vestibulitis, vulvodynia, dyspareunia, endometriosis in women prostatitis, chronic pelvic pain syndrome, urethral syndrome in men, radiation-induced cystitis, chemotherapy-induced cystitis, interstitial cystitis and overactive bladder in men and women.

## Patentansprüche

1. Pharmazeutische Zusammensetzung zur Verwendung bei der Behandlung oder Prophylaxe einer Erkrankung der unteren Harnwewge, enthaltend:
(a) 160 mg Lidocain pro Einzeldosis;
(b) 40000 Einheiten Heparin pro Einzeldosis;
(c) 336 mg Natriumbicarbonat pro Einzeldosis und
(d) 20 mg Natriumchlorid pro Einzeldosis,
so dass in einer Endeinzeldosis mit Volumen von 12 ml Lidocain mit 46 mM vorhanden ist, Heparin mit 3333 units/ml vorhanden ist, Natriumbicarbonat mit 0,33 M vorhanden ist und Natriumchlorid mit 28,5 mM vorhanden ist, wobei die Erkrankung der unteren Harnwege ausgewählt ist aus der Gruppe bestehend aus: bakterieller Zystitis, Pilz- /Hefe-Zystitis, Vulva-Vestibulitis, Vulvodynie, Dyspareunie, Endometriose bei Frauen, Prostatitis, chronischem pelvic pain Syndrom, urethralem Syndrom bei Männern, strahleninduzierter Zystitis, Chemotherapieinduzierter Zystitis, interstitieller Zystitis und überaktiver Blase bei Männern und Frauen.

2. Pharmazeutische Zusammensetzung zur Verwendung bei der Behandlung oder der Prophylaxe von Erkrankungen der unteren Harnwege, enthaltend:
(a) 200 mg Lidocain pro Einzeldosis;
(b) 40000 Einheiten Heparin pro Einzeldosis;
(c) 336 mg Natriumbicarbonat pro Einzeldosis und
(d) 20 mg Natriumchlorid pro Einzeldosis,
so dass in einer Endeinzeldosis mit Volumen von 13 ml, Lidocain mit 53 mM vorhanden ist, Heparin mit 3077 units/ml vorhanden ist, Natriumbicarbonat mit 0,305 M vorhanden ist und Natriumchlorid mit 26,3 mM vorhanden ist, wobei die Erkrankung der unteren Harnwege ausgewählt ist aus der Gruppe bestehend aus: bakterieller Zystitis, Pilz- /Hefe-Zystitis, Vulva-Vestibulitis, Vulvodynie, Dyspareunie, Endometriose bei Frauen, Prostatitis, chronischem pelvic pain Syndrom, urethralem Syndrom bei Männern, strahleninduzierter Zystitis, Chemotherapieinduzierter Zystitis, interstitieller Zystitis und überaktiver Blase bei Männern und Frauen.

3. Pharmazeutische Zusammensetzung zur Verwendung bei der Behandlung oder der Prophylaxe von Erkrankungen der unteren Harnwege, enthaltend:
(a) 240 mg Lidocain pro Einzeldosis;
(b) 40000 Einheiten Heparin pro Einzeldosis;
(c) 336 mg Natriumbicarbonat pro Einzeldosis und
(d) 20 mg Natriumchlorid pro Einzeldosis,
so dass in einer Endeinzeldosis mit Volumen von 14 ml Lidocain mit 64 mM vorhanden ist, Heparin mit 2857 units/ml vorhanden ist, Natriumbicarbonat mit 0,28 M vorhanden ist und Natriumchlorid mit 24,4 mM vorhanden ist, wobei die Erkrankung der unteren Harnwege ausgewählt ist aus der Gruppe bestehend aus: bakterieller Zystitis, Pilz- /Hefe-Zystitis, Vulva-Vestibulitis, Vulvodynie, Dyspareunie, Endometriose bei Frauen, Prostatitis, chronischem pelvic pain Syndrom, urethralem Syndrom bei Männern, strahleninduzierter Zystitis, Chemotherapieinduzierter Zystitis, interstitieller Zystitis und überaktiver Blase bei Männern und Frauen.

## Revendications

1. Composition pharmaceutique pour une utilisation dans le traitement ou la prévention d'un trouble des voies urinaires inférieures, comprenant :
(a) 160 mg de lidocaïne par dose unitaire ;
(b) 40 000 unités d'héparine par dose unitaire ;
(c) 336 mg de bicarbonate de sodium par dose unitaire ; et
(d) 20 mg de chlorure de sodium par dose unitaire ;
de sorte que, dans un volume final de dose unitaire de 12 ml, la lidocaïne est présente à 46 mM, l'héparine est présente à 3 333 unités/ml, le bicarbonate de sodium est présent à 0,33 M et le chlorure de sodium est présent à 28,5 mM, ledit trouble des voies urinaires inférieures étant choisi dans le groupe constitué de la cystite d'origine bactérienne, de la cystite ayant pour origine un champignon ou une levure, de la vestibulite vulvaire, de la vulvodynie, de la dyspareunie, de l'endométriose chez les femmes, de la prostatite, du syndrome de douleurs pelviennes chroniques, du syndrome urétral chez les hommes, de la cystite radio-induite, de la cystite induite par une chimiothérapie, de la cystite interstitielle et de la vessie hyperactive chez les hommes et les femmes.

2. Composition pharmaceutique pour une utilisation dans le traitement ou la prévention d'un trouble des voies urinaires inférieures, comprenant :
(a) 200 mg de lidocaine par dose unitaire ;
(b) 40 000 unités d'héparine par dose unitaire ;
(c) 336 mg de bicarbonate de sodium par dose unitaire ; et
(d) 20 mg de chlorure de sodium par dose unitaire ;
de sorte que, dans un volume final de dose de 13 ml, la lidocaine est présente à 53 mM, l'héparine est présente à 3 077 unités/ml, le bicarbonate de sodium est présent à 0,305 M et le chlorure de sodium est présent à 26,3 mM, ledit trouble des voies urinaires inférieures étant choisi dans le groupe constitué de la cystite d'origine bactérienne, de la cystite ayant pour origine un champignon ou une levure, de la vestibulite vulvaire, de la vulvodynie, de la dyspareunie, de l'endométriose chez les femmes, de la prostatite, du syndrome de douleurs pelviennes chroniques, du syndrome urétral chez les hommes, de la cystite radio-induite, de la cystite induite par une chimiothérapie, de la cystite interstitielle et de la vessie hyperactive chez les hommes et les femmes.

3. Composition pharmaceutique pour une utilisation dans le traitement ou la prévention d'un trouble des voies urinaires inférieures, comprenant :
(a) 240 mg de lidocaine par dose unitaire ;
(b) 40 000 unités d'héparine par dose unitaire ;
(c) 336 mg de bicarbonate de sodium par dose unitaire ; et
(d) 20 mg de chlorure de sodium par dose unitaire ;
de sorte que, dans un volume final de dose de 14 ml, la lidocaïne est présente à 64 mM, l'héparine est présente à 2 857 unités/ml, le bicarbonate de sodium est présent à 0,28 M et le chlorure de sodium est présent à 24,4 mM, ledit trouble des voies urinaires inférieures étant choisi dans le groupe constitué de la cystite d'origine bactérienne, de la cystite ayant pour origine un champignon ou une levure, de la vestibulite vulvaire, de la vulvodynie, de la dyspareunie, de l'endométriose chez les femmes, de la prostatite, du syndrome de douleurs pelviennes chroniques, du syndrome urétral chez les hommes, de la cystite radio-induite, de la cystite induite par une chimiothérapie, de la cystite interstitielle et de la vessie hyperactive chez les hommes et les femmes.
